# EUROPEAN PATENT SPECIFICATION

(11) **EP 0 803 550 B1**
(45) Date of publication and mention of the grant of the patent: **09.07.2003**
(21) Application number: 97105916.7
(22) Date of filing: 10.04.1997
(51) Int. Cl.: C09C 1/30, C09C 3/06, C09C 3/12, C09D 7/12, C09D 11/00, C08K 9/02, C08K 9/06, A61K 7/42, A61K 7/00

(54) **Coated SiO2 particles**
Beschichtete SiO2-Teilchen
Particules de SiO2 revêtues

(30) Priority: 22.04.1996 EP 96106278
(43) Date of publication of application: 29.10.1997
(73) Proprietor: MERCK PATENT GmbH, 64293 Darmstadt (DE)
(72) Inventor: Noguchi, Tamio, Iwaki-shi, Fukushima-ken (JP); Iwasa, Kazuhisa, Fukushima-ken (JP); Anselmann, Ralf, Dr., 64839 Münster (DE); Knapp, Martin, 64807 Dieburg (DE); Loch, Manuela, 55627 Merxheim (DE)

(56) References cited:
- EP-A- 0 172 730
- EP-A- 0 778 319
- US-A- 5 512 094
- DATABASE WPI Week 8618 Derwent Publications Ltd., London, GB; AN 86-116717 XP002105634 & JP 61 057653 A (NIPPON MENARD TESHOHIN ET AL.), 24 March 1986
- DATABASE WPI Week 9118 Derwent Publications Ltd., London, GB; AN 91-130214 XP002105635 & JP 03 070768 A (TORAY IND.) , 26 March 1991
- DATABASE WPI Week 9340 Derwent Publications Ltd., London, GB; AN 93-317707 XP002105636 & JP 05 230394 A (KAO), 7 September 1993

## Description

The invention relates to spherical SiO₂ particles coated with oxides of the elements titanium, iron or zircon, to a process for their preparation and to the use of the products obtained.

Spherical SiO₂ particles are known per se from the prior art. SiO₂ particles are obtained by hydrolytic polycondensation from alcoholate compounds, after which they are also obtained in the form of compact, monodisperse, spherical particles. The fundamental reaction conditions for the preparation of SiO₂ particles by hydrolytic polycondensation can be found, for example, in the publications by W. Stöber et al. in J. Colloid and Interface Science 26, 62 (1968) and 30, 568 (1969) and in US Patent 3,634,588. The particles thus prepared, however, often show large standard deviations in their particle diameter and have a certain porosity.

EP-A-0 172 730 discloses surface-stabilized porous silica particles having a discontinuous metal oxide layer over the silica, which may comprise an oxide of Zr, Fe or Ti.

Derwent abstract of JP 05-230994 discloses a composite filler for cosmetics, having a layer construction with a core of mica, talc, silica or alumina, the shape of the core is platelet or globular, further on a first layer of TiO₂ or ZrO₂ and a second top layer of ZuO, wherein the total thickness of both layers is from 15-150 nm and the weight ratio of first/second layer is 0.02 - 2.0.

For the preparation of highly monodisperse, nonporous, spherical SiO₂ particles which have a standard deviation of not more than 5%, reference is made to EP 0 216 278, which discloses a correspondingly oriented preparation process based on hydrolytic polycondensation. The key feature of this process, which is preferred for the preparation of the particles according to the present invention, is a two-stage procedure. In this procedure, hydrolytic polycondensation of tetraalkoxysilanes in aqueous-alkaline-ammoniacal medium is first used to form a sol or a suspension of primary particles, which are then brought by metered addition of further tetraalkoxysilane to the desired final size.

An appropriate process for the preparation of various metal oxides in the form of spherical particles with narrow particle size distribution is to be found in EP 0 275 688.

A corresponding two-stage process for the preparation of various metal oxides and also mixed oxides, which, furthermore, also have glycolic groups chemically bonded to the surface, is described in EP 0 391 447.

SiO₂ particles organically modified in this way can be used as tailor-made sorbents for chromatography. They are particularly suitable for use in reversed-phase chromatography. The use of these particles allows the separation of high molecular weight biomolecules, such as peptides, proteins or nucleic acids.

The subsequent coating of spherical SiO₂ particles with titanium dioxide is described in Japanese Published Specification No. 06-011 872. SiO₂ particles with a size of 0.5-50 µm are coated by suspending them in an aqueous titanyl sulfate solution and heating the suspension, the titanyl sulfate hydrolysing and the resulting titanium oxide hydrate being precipitated onto the SiO₂ particles. In this process, the entire surface is coated with titanium dioxide. The weight ratio of silicon dioxide to titanium dioxide is within the range from 10:90 to 90:10.

The size of the individual crystallites within the continuous TiO₂ coat is about 500 nm.

The coated SiO₂ particles are separated off, washed and dried in accordance with known methods. In order to increase their mechanical strength they are heated at from 500 to 900°C for from 30 minutes to 5 hours.

The product obtained possesses a high masking capacity and a screening effect towards ultraviolet radiation. It is used in cosmetology as a component of make-up formulations.

The high masking capacity of this product makes it unsuitable for use in sunscreen compositions. A sunscreen composition should possess a high absorption and reflection capacity for ultraviolet radiation but at the same time should be highly transparent to the visible component of sunlight, so that it is not visible on the skin.

The object of the present invention is to provide a product which consists of spherical SiO₂ particles coated at individual points with oxide particles of the elements titanium, iron or zircon. The product is to have a high absorption and reflection capacity for ultraviolet radiation while in contrast being highly transparent to visible light. Furthermore, the product should not have a tendency towards agglomeration, such a tendency being a known feature of conventional UV protection filters, for example ultrafine titanium dioxide.

A further object of the present invention is to provide a product which is suitable as filler in organic matrix materials, with the refractive index of the particles being adapted as a function of the application to the refractive index of the organic matrix.

It has, surprisingly, been found that, by means of a specific procedure, it is possible to coat spherical SiO₂ particles with a size of from 5 to 500 nm at individual points with oxide particles of the elements titanium, iron or zircon with a size of less than 60 nm to give highly transparent products which possess a high absorption and reflection capacity in the ultraviolet region.

The invention therefore provides spherical SiO₂ particles with a size of from 5 to 500 nm, preferably with a size of less than 100 nm, which are coated at individual points with oxide particles of the elements titanium, iron or zircon or mixtures of these methaloxides with a size of less than 60 nm, which coated particles are obtainable for example by adding a from 5 to 40% titanium tetrachloride solution at a metering rate of from 0.0005 to 0.5 mg of TiO₂ per minute and per m² surface area of the SiO₂ particles to an aqueous dispersion of the SiO₂ particles at a pH of from 1.3 to 2.5, preferably from 1.6 to 2.0, separating off the coated SiO₂ particles and subjecting them to drying and, if desired, calcination.

The invention additionally provides a process for the preparation of spherical SiO₂ particles with a size of from 5 to 500 nm, preferably less than 100 nm, which are coated at individual points with oxide particles of the elements titanium, iron or zircon with a size of less than 60 nm, which is characterized in that the SiO₂ particles are dispersed in deionized water at a temperature of from 50 to 90°C in a concentration of from 1 to 30% by weight, preferably 5-10% by weight, in case of a coating with TiO₂ particles an aqueous from 5 to 40% titanium salt solution is added at a pH of from 1.3 to 2.5, preferably from 1.6 to 2.0, at a metering rate of from 0.0005 to 0.5 mg of TiO₂ per minute and per m² surface area of the SiO₂ particles, the pH being kept constant by simultaneous addition of a base, the coated SiO₂ particles are separated off, washed with water and then with ethanol, are initially dried in air and are then dried in vacuo at from 70 to 125°C.

The invention also provides for the use of the particles according to the invention as sunscreen agents in cosmetic formulations and as filler in organic matrix materials. Furthermore they are used for pimenting paints, printing inks, plastics and coatings.

Specific embodiments of the invention-are comprised in the subclaims.

The spherical SiO₂ particles to be used as starting material are known per se from the prior art.

The fundamental reaction conditions for the preparation of SiO₂ particles by hydrolytic polycondensation can be found, for example, in the publications by W. Stöber et al. in J. Colloid and Interface Science 26, 62 (1968) and 30, 568 (1969) and in US Patent 3,634,588. The particles thus prepared, however, often show large standard deviations in their particle diameter and have a certain porosity.

For the preparation of highly monodisperse, nonporous, spherical SiO₂ particles which have a standard deviation of not more than 5%, reference is made to EP 0 216 278, which discloses a correspondingly oriented preparation process based on hydrolytic polycondensation. The key feature of this process, which is preferred for the preparation of the particles according to the present invention, is a two-stage procedure. In this procedure, hydrolytic polycondensation of tetraalkoxysilanes in aqueous-alkaline-ammoniacal medium is first used to form a sol or a suspension of primary particles, which are then brought by metered addition of further tetraalkoxysilane to the desired final size.

The grain size of the SiO₂ particles to be employed as starting material is dependent on the intended use of the SiO₂ particles according to the invention which are coated with oxide particles of the elements titanium, iron and zircon.

Particle sizes of between 5 nm and 500 nm are available. For the use of the products according to the invention as sunscreen agents in cosmetic formulations, for example, particle sizes of from 5 to 100 nm are employed.

For the use as filler in organic matrix materials, particle sizes of from 50 to 500 nm are employed. Depending on the choice of the oxides on which the inorganic particles are based, the refractive index of the products according to the invention can be tailored precisely to the refractive index of the organic matrix. Polymers or polymerizable systems comprising these particles can be used, for example, as embedding compositions for optical, electrooptical and optoelectrical components. Such embedding compositions exhibit an improved optical homogeneity. Light-emitting diodes produced with such compositions are distinguished inter alia by an enhanced light yield. Further details on this use of the products according to the invention are given in WO 93/25611.

The size of the TiO₂, Fe₂O₃ and ZrO₂ particles in the products according to the invention is below 60 nm.

The proportion of titanium dioxide, iron oxide or zircon oxide in the product according to the invention is from 20 to 75% by weight, preferably from 40 to 50% by weight.

The coated SiO₂ particles according to the invention are also produced on the following way: An aqueous solution of a metal salt, e.g. titanium tetrachloride, is heated to 60 °C with stirring. The obtained suspension of the metal oxide is added dropwise to a suspension of spherical SiO₂ particles. The pH is adjusted to 2,0 with 32 % NaOH solution with stirring and a silane coupling agent is added to the suspension. After 15 minutes the pH value is increased to 8,0 with 32 % NaOH solution and the suspension is stirred again for 10 minutes. After filtering, washing and drying the coated SiO₂ particles are powdered by using a blender and the obtained powder is claicinated at 700 °C for 5 minutes.

The products according to the invention can be aftercoated with organic and inorganic compounds by known methods.

By aftercoating with silanes or metal oxides it is possible to prevent the agglomeration of the monodisperse particles during the drying process. In the case where the products according to the invention are used as sunscreen agents, it is possible by aftercoating with iron oxide to adjust the colour to a defined skin shade. Aftercoating with zinc oxide increases the effectiveness of the product as a UV-A absorber.

When aftercoating with a silane of the general formula RₙSi(OX)₃, in which Rₙ is an alkyl group having 1 to 18 carbon atoms and X is an alkyl group having 1 or 2 carbon atoms, from 0.02 to 2% by weight of the silane, calculated as SiO₂, are applied to the product according to the invention. Preference is given to the use of CH₃Si(OMe)₃,

Preferred metal oxides used for aftercoating are zinc oxide, iron oxide, zirconium oxide and cerium oxide.

From SEM micrographs of the products according to the invention with two different particle sizes, 250 and 50 nm, it is evident that the titanium dioxide is arranged at individual points on the surface of the SiO₂ particles and does not form, as is the case with known products, for example according to JP 06-011 872 (Kokoku), a continuous coat.

The concentration of the additionally applied metal oxides is from 1 to 100% by weight, preferably 10% by weight, based on the content of TiO₂.

For the process according to the invention for the preparation of the SiO₂ particles coated at individual points with TiO₂ particles, it is essential to avoid an excess of titanium salt. This is achieved in that the quantity of titanium salt supplied to the hydrolysis per unit time is only that necessary for uniform formation of the TiO₂ particles. This effect is best achieved if the hydrolysis is carried out at a temperature which is as constant as possible and with approximately constant pH.

The from 5 to 40% titanium tetrachloride solution is added at a metering rate of from 0.0005 to 0.5 mg of TiO₂ per m² surface area of the SiO₂ particles per minute to an aqueous dispersion of the SiO₂ particles at a pH of from 1.3 to 2.5, preferably from 1.6 to 2.0.

Since the different fractions of SiO₂ particles possess different diameters and therefore different surface areas, the values are different for each particle size when equal quantities are employed.

| Particle ⌀ in nm | Surface area m²/g | Min. quantity of TiO₂ per m² per minute | Max. quantity of TiO₂ per m² per minute |
|---|---|---|---|
| 1000 | 3 | 0.02 mg | 0.5 mg |
| 500 | 6 | 0.01 mg | 0.25 mg |
| 250 | 12 | 0.005 mg | 0.15 mg |
| 100 | 30 | 0.002 mg | 0.05 mg |
| 50 | 60 | 0.001 mg | 0.025 mg |
| 25 | 120 | 0.0005 mg | 0.012 mg |

The TiCl₄ solution used preferably has a concentration of 15% by weight of TiCl₄ with a density of 1.123 g/ml. The concentration range of the TiCl₄ solutions to be used extends from 5 to 40% by weight.

In accordance with the process of the invention, it is possible to offer the surfaces to be coated, per unit time, only a small quantity of titanium salt such that all of the titanium dioxide hydrate is able to deposit on the surfaces, and no freely mobile by-products can be formed in the dispersion. Further details are described in US 3,553,001.

The SiO₂ particles according to the invention, coated with titanium dioxide, iron oxide or zircon oxide, can, depending on their intended use, be coated with further metal oxides or with organic compounds, for example silanes, by known methods.

Coating with zinc oxide is likewise carried out by known methods. The zinc oxide hydrate is precipitated by hydrolysis of zinc chloride using an ammonium complex at a pH of from 11 to 12. The SiO₂ particles according to the invention which are coated with TiO₂ particles are suspended at room temperature in deionized water, the pH is adjusted to 11 - 12 with ammonia solution, and a prepared zinc chloride solution is then added. By slow heating of the suspension the ammonia is driven off, the pH of the suspension falls and the zinc oxide hydrate is slowly precipitated. The particles coated with zinc oxide hydrate are separated off, washed with water and then with ethanol, initially dried in air and then dried in vacuo at from 70 to 125°C.

The zinc oxide hydrate can also be precipitated by hydrolysing zinc oxalate or zinc chloride.

Aftercoating of the particles according to the invention with iron(III) oxide is likewise carried out by known methods; the same applies to aftercoating with zirconium oxide.

In the case of aftercoating with iron(III) oxide, an iron(III) chloride solution is metered at a temperature of from 60 to 90°C and at a pH of from 2.5 to 4.5 into an aqueous suspension of the SiO₂ particles according to the invention, coated with titanium dioxide. The pH is kept constant by simultaneous addition of 32% sodium hydroxide solution.

The particles coated with iron(III) oxide are separated off, washed with water and then with ethanol, initially dried in air and then dried in vacuo at from 70 to 125°C.

The pigments according to the invention are dried at temperatures from 110 to 125 °C under normal pressure or from 70 to 125 °C in vacuo. Dependent on the purpose the pigments can be additionally calcined at temperatures from 300 °C to 900 °C for from 5 to 60 minutes.

The pigments according to the invention are used for pigmenting paints, printing inks, plastics, coatings or as sunscreen agent in cosmetic formulations.

When the pigment according to the invention is used as sunscreen agent in cosmetic formulations, the pigment is incorporated into the formulation in a concentration of up to 5% by weight.

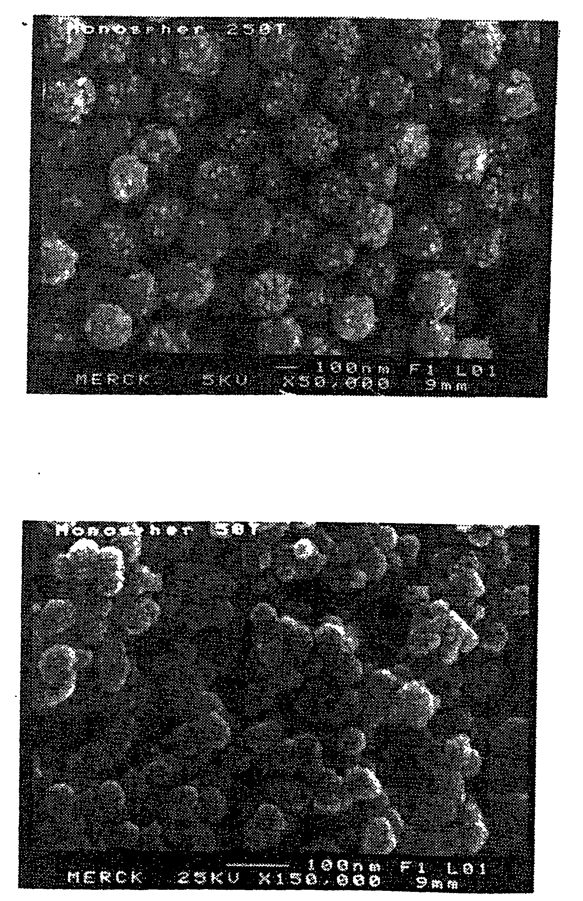

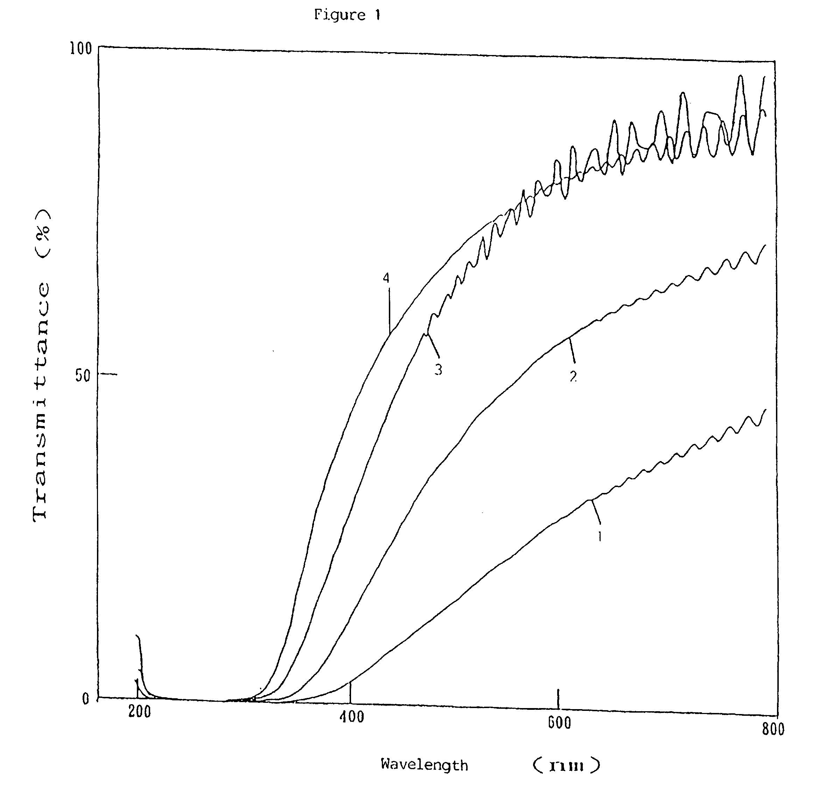

Figure 1 compares two embodiments of the pigment according to the invention with known sunscreen agents with respect to their transparency in the wavelength range from 220 to 800 nm. For this comparison, the products were incorporated into VS medium of Dainichiseika Co. Ltd. in a concentration of 1.5% by weight, and the transparency was measured.

The VS medium contains a copolymer of vinyl chloride and vinyl acetate as main resin and xylene in a concentration of from 30 to 40 $ and cyclohexanone in a concentration of from 50 to 60 %

Plot 1 shows the transparency of an ultrafine titanium dioxide, while plot 2 shows the transparency of an ultrafine titanium dioxide coated with iron oxide. Plot 3 shows the transparency of a pigment according to the invention having the composition 27% SiO₂, 53% TiO₂, 20% Fe₂O₃. Plot 4 4 shows the transparency of a pigment according to the invention having the composition 31% SiO₂ and 69% TiO₂.

The plots show that, in the region of visible light, the pigments according to the invention possess a substantially higher transparency than ultrafine titanium dioxide. This means that ultrafine titanium dioxide causes a white film to appear on the hand, whereas the pigments according to the invention remain invisible on the skin owing to their high transparency.

Table 1 compares the transparency and absorbance of the 4 abovementioned pigments at identical and at different concentrations in the formulation.

**Table 1**

| Pigment No. | Concentr % by weight | Transmittance (%) | | | Absorbance | |
|---|---|---|---|---|---|---|
| | | Visible light | UV-A | UV-B | UV-A | UV-B |
| | | (550 nm) | (380 nm) | (300nm) | (380nm) | (300 nm) |
| 1 | 1.5 | 22 | 1 | 0 | 2.0 | 3.7 |
| 2 | 1.5 | 76 | 34 | 0 | 0.5 | 2.7 |
| 3 | 1.5 | 49 | 6 | 0 | 1.2 | 3.0 |
| 4 | 1.5 | 75 | 19 | 0 | 0.7 | 2.8 |
| 5 | 0.75 | 66 | 12 | 1 | 0.9 | 2.0 |
| 6 | 3.0 | 64 | 12 | 0 | 0.9 | 3.4 |
| 7 | 1.0 | 63 | 17 | 2 | 0.7 | 1.7 |
| 8 | 2.0 | 62 | 8 | 0 | 1.2 | 3.3 |
| Pigments Nos. 1 and 5: Ultrafine TiO₂ Pigments Nos. 2 and 6: Invention (31% SiO₂, 69% TiO₂) Pigments Nos. 3 and 7: Ultrafine TiO₂ + Fe₂O₃ Pigments Nos. 4 and 8: Invention (27% SiO₂, 53% TiO₂, 20% Fe₂O₃) | | | | | | |

The table shows that pigment No. 2 according to the invention possesses a transparency at 550 nm which is approximately 3.5 times higher than the transparency of ultrafine titanium dioxide (pigment No. 1).

To establish approximately the same transparency, relative to ultrafine titanium dioxide, 4 times the quantity of the pigment according to the invention can be added to a cosmetic formulation. This means that, with the pigment according to the invention, it is possible to achieve a markedly higher light protection effect.

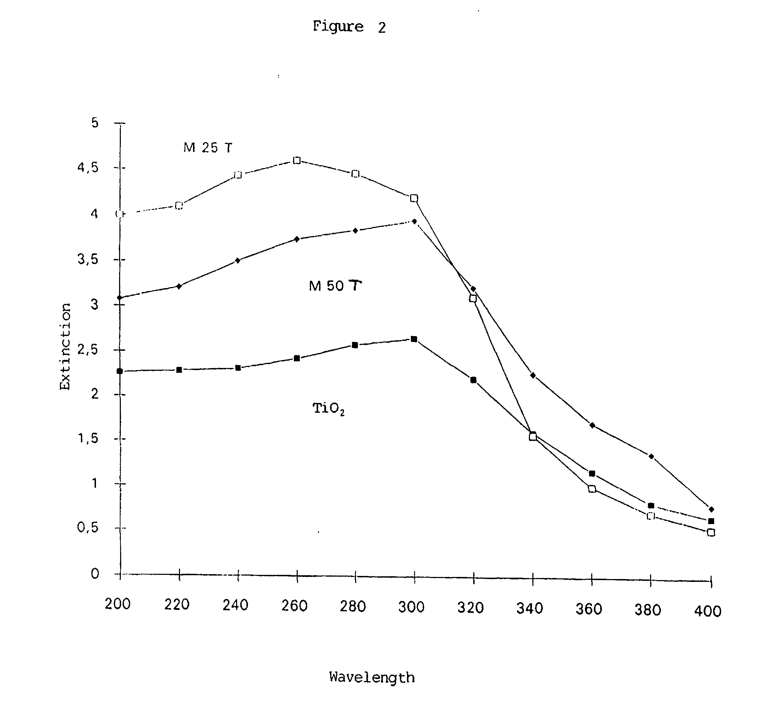

Figure 2 compares two embodiments of the pigment according to the invention with a known sunscreen agent (ultrafine TiO₂) with respect to their extinction in the wavelength range from 200 to 400 nm. The SiO₂ particles coated with TiO₂ (particles sizes 25 and 50 nm) show a significantly higher extinction (shielding capability) in the wavelength range from 200 to 320 nm. However, in the range of the visible light the pigments of the invention show the same extinction (transparency) as the ultrafine TiO₂.

According to the invention SiO₂ as a core particle for newly pigments is coated with TiO₂ particles. Due to TiO₂ particles contact the core SiO₂ particles directly, TiO₂ particles can perform as if they were single particle keeping. Thereby this fine particle of TiO₂ coated SiO₂ pigment has superior transmittance and great UV ray shielding capability. This newly developed pigment can be possible to contain highly in a sunscreen. products because of those superior transparency, so it will be able to make the excellent sunscreen which has high UV-ray shielding capability.

In addition, the Fe₂O₂ type pigment is treated with iron oxide on the surface of TiO₂ coated colloidal silica particles. For UV-A&B range Fe₂O₂ has also shielding capability, so this iron oxide type pigment has higher UV-A&B shielding capability compared with TiO₂ type pigment because of the efficacy of combination TiO₂ and Fe₂O₂. With the addition the pigment coloring to skintone can be get by the content of iron oxide. Sunscreen products with several skin tone in cosmetics can be

produced. The examples which follow are intended to describe the invention in more detail without limiting it.

### Example 1

3000 g of an aqueous dispersion of SiO₂ particles with a size of 250 nm (5% by weight SiO₂) are heated to 75°C with stirring. 198 g of 60% TiCl₄ solution are diluted with 141 g of deionized water and metered at a metering rate of 1.5 ml/min into the dispersion. By simultaneous addition of 32% NaOH solution, the pH is kept constant at 2.2. After 60 minutes, the metering rate of the TiCl₄ solution is increased to 3 ml/min. Metered addition of the TiCl₄ solution is complete after 110 minutes. The reaction mixture is then stirred for a further 15 minutes and neutralized with NaOH solution, and the solid is separated off, washed free from salt, then washed with 0.5 1 of ethanol and, after initial drying in air, is dried overnight in a vacuum drying cabinet at from 70 to 75°C. The TiO₂ content of the pigment is 25% by weight.

### Example 2

3000 g of an aqueous dispersion of SiO₂ particles with a size of 250 nm (5% by weight SiO₂) are heated to 75°C with stirring. 350 g of iron(III) chloride solution (15% by weight Fe, Merck Art. 5513) are diluted with 306 g of deionized water to an iron content of 8% by weight. The solution is metered into the dispersion at 0.5 ml/min. After 10 minutes, the rate is increased to 1.0 ml/min and, after a further 10 minutes, to 1.5 ml/min. This rate is maintained until the end of coating. By simultaneous addition of 32% sodium hydroxide solution, the pH is kept constant at between 3.2 and 3.3. Addition is complete after about 6 hours. After the end of the addition of FeCl₃, stirring is continued for 15 minutes and the mixture is then neutralized with the NaOH solution. The solid is separated off, washed free from salt, washed with 0.5 l of ethanol and finally, after initial drying in air, is dried overnight in a vacuum drying cabinet at 70-75°C. The Fe₂O₃ content of the finished pigment, based on oxides (SiO₂ and Fe₂O₃), is 33% by weight.

### Example 3

SiO₂ particles with a size of 250 nm are coated with TiO₂ in accordance with Example 1. The dispersion is then adjusted to a pH of 3.2 using NaOH solution. 11.5 g of iron(III) chloride solution (15% by weight Fe, Merck Art. 5513) are diluted with 46.9 g of water to an iron content of 3% by weight and metered at a rate of 0.5 ml/min into the abovementioned dispersion . After 60 minutes, the rate of metering is increased to 1.0 ml/min. Addition is complete after about 90 minutes. During the addition of the iron chloride solution, the pH is held between 3.2 and 3.3 by simultaneous addition of NaOH solution. The reaction mixture is then stirred for a further 15 minutes and neutralized with NaOH solution, and the solid is separated off, washed free from salt, then washed with 0.5 l of ethanol and, after initial drying in air, is dried overnight in a vacuum drying cabinet at from 70 to 75°C. The iron content of the pigment is 5% by weight Fe₂O₃, based on the TiO₂ content of the pigment.

### Example 4

2400 g of an aqueous dispersion of SiO₂ particles with a size of 250 nm (5% by weight SiO₂) are heated to 75°C with stirring. 160 g of 60% TiCl₄ solution are diluted with 112 g of deionized water, and 6.8 g of zinc chloride are added to the solution with stirring and dissolved. This solution is metered at a rate of 1.2 ml/min into the abovementioned dispersion, the pH being kept constant at from 2.2 to 2.3 by simultaneous addition of NaOH solution. After 60 minutes, the rate of metering is increased to 2.4 ml/min. Addition is complete after about 110 minutes. The reaction mixture is subsequently stirred for a further 15 minutes and neutralized over the course of 25 minutes with the aid of an NaOH solution. To ensure complete precipitation of the zinc hydroxide, the dispersion is subsequently stirred for 30 minutes and then allowed to cool to room temperature. The solid is then separated off, washed free from salt, then washed with 0.5 l of ethanol and, after initial drying in air, is dried overnight in a vacuum drying cabinet at from 70 to 75°C. The zinc oxide content of the pigment is 10% by weight, based on the TiO₂ content of the pigment.

### Example 5

2400 g of an aqueous dispersion of SiO₂ particles with a size of 250 nm (5% by weight SiO₂) are heated to 75°C with stirring. 160 g of 60% TiCl₄ solution are diluted with 112 g of deionized water and metered at a rate of 1.2 ml/min into the abovementioned dispersion, the pH being kept constant at from 2.2 to 2.3 by simultaneous addition of NaOH solution. After 60 minutes, the rate of metering is increased to 2.4 ml/min. Addition is complete after about 110 minutes. The reaction mixture is subsequently stirred for a further 15 minutes and neutralized over the course of 25 minutes with the aid of an NaOH solution. Subsequently, a zinc chloride solution and an oxalic acid solution are metered, simultaneously but separately from one another, each at a rate of 1.2 ml/min into the neutralized reaction mixture, the pH being kept constant at 7 by simultaneous addition of an NaOH solution. The zinc chloride solution contains 6.8 g of zinc chloride in 61 g of water. The oxalic acid solution contains 6.3 g of oxalic acid in 61 g of water. Addition is complete after about 55 minutes. To ensure complete precipitation of the zinc oxide, the dispersion is subsequently stirred and then allowed to cool to room temperature. The solid is separated off, washed free from salt, then washed with 0.5 l of ethanol and, after initial drying in air, is dried overnight in a vacuum drying cabinet at from 70 to 75°C. The zinc oxide content of the pigment is 10% by weight, based on the TiO₂ content of the pigment.

### Example 6

3000 g of an aqueous dispersion of SiO₂ particles with a size of 250 nm (5% by weight SiO₂) are heated to 75°C with stirring.

198 g of 60% TiCl₄ solution are diluted with 142 g of deionized water and metered at a metering rate of 1.5 ml/min into the dispersion. By simultaneous addition of 32% NaOH solution, the pH is kept constant at 2.2. After 60 minutes, the metering rate of the TiCl₄ solution is increased to 3 ml/min. Metered addition of the TiCl₄ solution is complete after 110 minutes. The reaction mixture is then stirred for a further 15 minutes and neutralized with NaOH solution.

Using 25% ammonia solution, the pH of the dispersion is adjusted to 11 - 12, and a zinc chloride solution consisting of 8.4 g of zinc chloride in 162 g of deionized water is added. By slow heating of the dispersion, the ammonia is driven off and the zinc hydroxide is precipitated. The precipitation end point is reached when the addition of dilute hydrochloric acid to a filtered sample of the dispersion no longer produces a precipitate. After cooling to room temperature, the solid is worked up in accordance with Example 5.

The zinc oxide content of the pigment is 10% by weight, based on the TiO₂ content.

### Example 7

66 g (20 g as SiO₂) of colloidal silica sol, type ST-S, with a size distribution of 5 to 9 nm in 500 ml of deionized water are heated to 80°C with stirring. The pH is rapidly adjusted to 1.6 with conc. HCl. Then 256 ml (45 g as TiO₂) of TiCl₄ solution (418 g/l) are metered in over about 7 hours, while maintaining the pH at 1.6 using 32% NaOH solution. After stirring the suspension for 15 minutes, the pH is adjusted to 3.0 with 32% NaOH, and 3.3 g of silane coupling agent, CH₃Si(OMe)₃, in 50 ml of deionized water are fed into the reactor, and the suspension is held for 15 minutes. The pH is increased to 8.0 with 32% NaOH and the suspension is stirred again for 10 minutes. After filtering, washing and drying, the pigment (5 g) is calcined at a temperature of 700°C for 5 minutes.

### Example 8

The reaction process is the same as in Example 7 until the step of metered addition of TiCl₄. At this point, the pH is adjusted to 1.9 using 32% NaOH, and a solution of 33 g of FeCl₃.6H₂O in 100 ml of deionized water is metered in over 2 hours, maintaining a pH of 1.9 using 32% NaOH solution. After stirring the suspension for 15 minutes, the pH is adjusted to 3.0 with 32% NaOH, and then 4.2 g of silane coupling agent in 50 ml of deionized water are fed into the reactor. The suspension is held for 15 minutes. The pH is increased to 8.0 with 32% NaOH and the suspension is stirred again for 10 minutes. After filtering, washing and drying, the pigment (5 g) is calcined at a temperature of 700°C for 5 minutes.

### Example 9

| Raw Materials | | |
|---|---|---|
| A: | Liquid paraffin | 38.0 % |
| | Cetanol | 1.5 |
| | Beeswax | 6.0 |
| | Stearic acid | 20.0 |
| | POE (5,5) Cetyl ether | 1.5 |
| | Sorbitan monostearate | 2.5 |
| B: | 10 % NaOH | 1.0 |
| | DI-water | 31.5 |
| C: | Glycerin | 6.0 |
| | TiO₂ | 5.0 |
| | SiO₂ coated TiO₂ | 5.0 |

### Preparation

TiO₂ and SiO₂ coated TiO₂ are dispersed in glycerin. A and B group are heated up seperately at 75 °C, then the two mixtures are emulsified by high speed stirring. Lastly, mix the C group is mixed with emulsified A & B mixture at 50 °C.

### Example 10

An aqueous solution of titanium tetrachloride prepared from 50 ml of titanium tetrachloride and 500 ml of water (2,18 mol/l TiO₂) is heated to 60 °C with stirring. To the white TiO₂ suspension obtained is added dropwise 40 g (12 g as SiO₂) of colloidal silica sol, type XL, with a size distribution of 40 to 50 nm. The pH is increased to 2.0 with 32 % NaOH solution with stirring, and 2 g of CH₃Si(OMe)₂ (silane coupling agent) in 50 ml of deionized water are added into the suspension, and the suspension is held for 15 minutes. The pH is increased to 8,0 with 32 % NaOH solution and the suspension is stirred again for 10 minutes. After filtering, washing and drying the dried product is powdered by using a blender and the resulting powder (5 g ) is calcined at 700 °C for 5 minutes.

## Claims

1. Spherical SiO₂ particles with a size of from 5 to 500 nm coated at individual points with metal oxide particles with a size of less than 60 nm.

2. SiO₂ particles according to Claim 1, **characterized in that** the metal oxide is TiO₂, Fe₂O₃, ZrO₂ or mixtures of these metal oxides.

3. SiO₂ particles according to Claims 1 and 2, **characterized in that** the metal oxide content is from 20 to 75% by weight.

4. SiO₂ particles according to at least one of Claims 1 to 3, **characterized in that** the SiO₂ particles coated with metal oxides are aftercoated with inorganic and/or organic compounds.

5. SiO₂ particles according to Claim 4, **characterized in that** the organic compounds are silanes of the general formula RₙSi(OX)₃, in which Rₙ is an alkyl group having 1 to 18 carbon atoms and X is an alkyl group having 1 or 2 carbon atoms.

6. SiO₂ particles according to Claim 5, **characterized in that** the silane is CH₃Si(OMe)₃.

7. SiO₂ particles according to Claim 4, **characterized in that** the inorganic compounds are metal oxides.

8. SiO₂ particles according to Claim 7, **characterized in that** the metal oxides are zinc oxide, iron oxide or zirconium oxide.

9. Process for the preparation of spherical SiO₂ particles with a size of from 5 to 500 nm which are coated at individual points with metal oxide particles with a size of less than 60 nm, where an aqueous solution of titanium tetrachloride is heated to 60 °C with stirring, the obtained TiO₂ suspension is added dropwise to a suspension of spherical SiO₂ particles, the pH is adjusted to 2,0 with 32 % NaOH solution with stirring and a silane coupling agent is added to the suspension, after 15 minutes the pH value is increased to 8,0 with 32 % NaOH solution and the suspension is stirred again for 10 minutes, after filtering, washing and drying the coated SiO₂ particles are powdered by using a blender and the obtained powder is calcined at 700 °C for 5 minutes.

10. Process for the preparation of spherical SiO₂ particles with a size of from 5 to 500 nm which are coated at individual points with TiO₂ particles with a size of less than 60 nm, where spherical SiO₂ particles are dispersed in deionized water at a temperature of from 50 to 90°C in a concentration of from 1 to 30% by weight, an aqueous 5 to 40% titanium salt solution is added at a pH of from 1.3 to 2.5 at a metering rate of from 0.0005 to 0.5 mg of TiO₂ per minute and per m² surface area of the SiO₂ particles, the pH being kept constant by simultaneous addition of a base, the coated SiO₂ particles are separated off, washed with water and then with ethanol, are initially dried in air and are then dried in vacuo at from 70 to 125°C, and then, if desired, are calcined.

11. Process according to Claim 10, **characterized in that** the coated SiO₂ particles are calcined at 300-900°C for from 5 to 60 minutes.

12. Process according to Claims 10 and 11, **characterized in that** the SiO₂ particles coated with metal oxides, without intermediate drying or thereafter, are aftercoated with inorganic and/or organic compounds.

13. Process according to at least one of Claims 10 to 12, **characterized in that** the organic compounds used are silanes of the general formula RₙSi(OX)₃, in which Rₙ is an alkyl group having 1 to 18 carbon atoms and X is an alkyl group having 1 or 2 carbon atoms.

14. Process according to Claim 13, **characterized in that** the silane used is CH₃Si(OMe)₃.

15. Process according to Claim 12, **characterized in that** the inorganic compounds used are metal oxides.

16. Process according to Claim 15, **characterized in that** the metal oxides are zinc oxide, iron oxide or zirconium oxide.

17. Use of the SiO₂ particles according to at least one of Claims 1 1 to 8 for pigmenting paints, printing inks, plastics and coatings or as sunscreen agents.

18. Cosmetic formulation comprising SiO₂ particles according to at least one of Claims 1 to 8 in a proportion of up to 5% by weight.

## Patentansprüche

1. Sphärische SiO₂-Teilchen mit einer Größe von 5 bis 500 nm und einer punktuellen Beschichtung mit Metalloxidteilchen mit einer Größe von weniger als 60 nm.

2. SiO₂-Teilchen nach Anspruch 1, **dadurch gekennzeichnet, daß** es sich bei dem Metalloxid um TiO₂, Fe₂O₃, ZrO₂ oder deren Mischungen handelt.

3. SiO₂-Teilchen nach Anspruch 1 und 2, **dadurch gekennzeichnet, daß** der Metalloxidgehalt bei 20 bis 75 Gew.-% liegt.

4. SiO₂-Teilchen nach mindestens einem der Ansprüche 1 bis 3, **dadurch gekennzeichnet, daß** die mit Metalloxiden beschichteten SiO₂-Teilchen mit anorganischen und/oder organischen Verbindungen nachbeschichtet sind.

5. SiO₂-Teilchen nach Anspruch 4, **dadurch gekennzeichnet, daß** es sich bei den organischen Verbindungen um Silane der allgemeinen Formel RₙSi(OX)₃ handelt, wobei Rₙ eine Alkylgruppe mit 1 bis 18 Kohlenstoffatomen und X eine Alkylgruppe mit 1 oder 2 Kohlenstoffatomen darstellt.

6. SiO₂-Teilchen nach Anspruch 5, **dadurch gekennzeichnet, daß** es sich bei dem Silan um CH₃Si(OMe)₃ handelt.

7. SiO₂-Teilchen nach Anspruch 4, **dadurch gekennzeichnet, daß** es sich bei den anorganischen Verbindungen um Metalloxide handelt.

8. SiO₂-Teilchen nach Anspruch 7, **dadurch gekennzeichnet, daß** es sich bei den Metalloxiden um Zinkoxid, Eisenoxid oder Zirkoniumoxid handelt.

9. Verfahren zur Herstellung sphärischer SiO₂-Teilchen mit einer Größe von 5 bis 500 nm und einer punktuellen Beschichtung mit Metalloxidteilchen mit einer Größe von weniger als 60 nm, bei dem man eine wäßrige Lösung von Titantetrachlorid unter Rühren auf 60°c erhitzt, die dabei entstandene TiO₂-Suspension einer Suspension von sphärischen SiO₂-Teilchen zutropft, den pH-Wert unter Rühren mit 32%iger NaOH-Lösung auf 2,0 einstellt und die Suspension mit einem Kupplungsreagens aus der Gruppe der Silane versetzt, nach 15 Minuten den pH-Wert mit 32%iger NaOH-Lösung auf 8,0 erhöht und die Suspension weitere 10 Minuten lang rührt, die nach Abfiltrieren, Nachwaschen und Trocknen erhaltenen beschichteten SiO₂-Teilchen im Mixer pulverisiert und das erhaltene Pulver 5 Minuten lang bei 700°C glüht.

10. Verfahren zur Herstellung von sphärischen SiO₂-Teilchen mit einer Größe von 5 bis 500 nm und einer punktuellen Beschichtung mit TiO₂-Teilchen mit einer Größe von weniger als 60 nm, bei dem man sphärische SiO₂-Teilchen in vollentsalztem Wasser bei einer Temperatur von 50 bis 90°C 1 bis 30 gew.-%ig dispergiert, eine wäßrige 5 bis 40%ige Titansalzlösung bei einem pH-Wert von 1,3 bis 2,5 zudosiert, wobei die Dosiergeschwindigkeit 0,0005 bis 0,5 mg TiO₂ pro Minute und m² Oberfläche der SiO₂-Teilchen beträgt und der pH-Wert durch gleichzeitige Zugabe einer Base konstant gehalten wird, die beschichteten SiO₂-Teilchen abtrennt, zunächst mit Wasser und dann mit Ethanol nachwäscht, zunächst an der Luft und dann unter Vakuum bei 70 bis 125°C trocknet und gegebenenfalls anschließend glüht.

11. Verfahren nach Anspruch 10, **dadurch gekennzeichnet, daß** man die beschichteten SiO₂-Teilchen 5 bis 60 Minuten lang bei 300-900°C glüht.

12. Verfahren nach Anspruch 10 und 11, **dadurch gekennzeichnet, daß** man die mit Metalloxiden beschichteten SiO₂-Teilchen nach oder ohne Zwischentrocknung mit anorganischen und/oder organischen Verbindungen nachbeschichtet.

13. Verfahren nach mindestens einem der Ansprüche 10 bis 12, **dadurch gekennzeichnet, daß** man als organische Verbindungen Silane der allgemeinen Formel RₙSi(OX)₃ einsetzt, wobei Rₙ eine Alkylgruppe mit 1 bis 18 Kohlenstoffatomen und X eine Alkylgruppe mit 1 oder 2 Kohlenstoffatomen darstellt.

14. Verfahren nach Anspruch 13, **dadurch gekennzeichnet, daß** man als Silan CH₃Si(OMe)₃ einsetzt.

15. Verfahren nach Anspruch 12, **dadurch gekennzeichnet, daß** man als anorganische Verbindungen Metalloxide einsetzt.

16. Verfahren nach Anspruch 15, **dadurch gekennzeichnet, daß** man als Metalloxide Zinkoxid, Eisenoxid oder Zirkoniumoxid einsetzt.

17. Verwendung der SiO₂-Teilchen gemäß mindestens einem der Ansprüche 1 bis 8 zur Pigmentierung von Anstrichmitteln, Druckfarben, Kunststoffen und Lacken oder als Sonnenschutzmittel.

18. Kosmetikformulierung, enthaltend SiO₂-Teilchen gemäß mindestens einem der Ansprüche 1 bis 8 in einem Anteil von bis zu 5 Gew.-%.

## Revendications

1. Particules de SiO₂ sphériques ayant une taille de 5 à 500 nm revêtues au niveau de points individuels de particules d'oxyde métallique ayant une taille inférieure à 60 nm.

2. Particules de SiO₂ selon la Revendication 1, **caractérisées en ce que** l'oxyde métallique est du TiO₂, du Fe₂O₃, du ZrO₂ ou des mélanges de ces oxydes métalliques.

3. Particules de SiO₂ selon la Revendication 1 ou la Revendication 2, **caractérisées en ce que** la teneur en oxyde métallique est de 20 à 75 % en poids.

4. Particules de SiO₂ selon, au moins l'une des Revendications 1 à 3, **caractérisées en ce que** les particules de SiO₂ revêtues avec les oxydes métalliques sont ensuite revêtues avec des composés inorganiques et/ou organiques.

5. Particules de SiO₂ selon la Revendication 4, **caractérisées en ce que** les composés organiques sont des silanes de formule générale RₙSi(OX)₃, dans laquelle Rₙ est un groupe alkyle ayant de 1 à 18 atomes de carbone et X est un groupe alkyle ayant 1 ou 2 atomes de carbone.

6. Particules de SiO₂ selon la Revendication 5, **caractérisées en ce que** le silane est du CH₃Si (OMe)₃.

7. Particules de SiO₂ selon la Revendication 4, **caractérisées en ce que** les composés inorganiques sont des oxydes métalliques.

8. Particules de SiO₂ selon la Revendication 7, **caractérisées en ce que** les oxydes métalliques sont de l'oxyde de zinc, de l'oxyde de fer ou de l'oxyde de zirconium.

9. Procédé de préparation de particules de SiO₂ sphériques ayant une taille allant de 5 à 500 nm qui sont revêtues au niveau de points individuels de particules d'oxyde métallique ayant une taille inférieure à 60 nm, dans lequel on chauffe une solution aqueuse de tétrachlorure de titane à 60°C tout en mélangeant, la suspension de TiO₂ obtenue est ajoutée goutte à goutte dans une suspension de particules de SiO₂ sphériques, le pH est ajusté à 2,0 avec une solution de NaOH à 32 % tout en mélangeant et un agent couplant de silane est ajouté à la suspension, après 15 minutes la valeur de pH est augmentée à 8,0 avec une solution de NaOH à 32 % et la suspension est de nouveau mélangée pendant 10 minutes et après filtration, lavage et séchage, les particules de SiO₂ revêtues sont mises en poudre au moyen d'un mélangeur et la poudre obtenue est calcinée à 700°C pendant 5 minutes.

10. Procédé de préparation de particules de SiO₂ sphériques ayant une taille allant de 5 à 500 nm qui sont revêtues au niveau de points individuels avec des particules de TiO₂ ayant une taille inférieure à 60 nm, dans lequel les particules de SiO₂ sphériques sont dispersées dans de l'eau désionisée à une température allant de 50 à 90°C à une concentration allant de 1 à 30 % en poids, une solution aqueuse à 5 - 40 % de sel de titane est ajoutée à un pH allant de 1,3 à 2,5 à une taux d'addition allant de 0,0005 à 0,5 mg de TiO₂ par minute et par m² de superficie des particules de SiO₂, le pH étant maintenu constant par l'addition simultanée d'une base, les particules de SiO₂ revêtues sont séparées, lavées à l'eau puis à l'éthanol et sont tout d'abord séchées à l'air, puis sont séchées sous vide à une température allant de 70 à 125°C, et ensuite, si on le souhaite, elles sont calcinées.

11. Procédé selon la Revendication 10, **caractérisé en ce que** les particules de SiO₂ revêtues sont calcinées à 300 - 900°C pendant un temps allant de 5 à 60 minutes.

12. Procédé selon les Revendications 10 et 11, **caractérisé en ce que** les particules de SiO₂ revêtues d'oxydes métalliques, sans chauffage intermédiaire ou postérieure, sont ensuite revêtues de composés inorganiques et/ou organiques.

13. Procédé selon au moins l'une des Revendications 10 à 12, **caractérisé en ce que** les composés organiques utilisés sont des silanes ayant la formule générale RₙSi(OX)₃, dans laquelle Rₙ est un groupe alkyle ayant de 1 à 18 atomes de carbone et X est un groupe alkyle ayant 1 ou 2 atomes de carbone.

14. Procédé selon la Revendication 13, **caractérisé en ce que** le silane utilisé est du CH₃Si (OMe)₃.

15. Procédé selon la Revendication 12, **caractérisé en ce que** les composés inorganiques utilisés sont des oxydes métalliques.

16. Procédé selon la Revendication 15, **caractérisé en ce que** les oxydes de métal sont de l'oxyde de zinc, de l'oxyde de fer ou de l'oxyde de zirconium.

17. Utilisation de particules de SiO₂ selon l'une au moins des Revendications 1 à 8 pour pigmenter des peintures, des encres d'impression, des plastiques et des revêtements, ou en tant qu'agents d'écran solaire.

18. Formulation cosmétique comprenant des particules de SiO₂ selon l'une au moins des Revendications 1 à 8 dans une proportion allant jusqu'à 5 % en poids.
